# EUROPEAN PATENT SPECIFICATION

(11) **EP 2 091 578 B1**
(45) Date of publication and mention of the grant of the patent: **10.01.2018**
(21) Application number: 07871263.5
(22) Date of filing: 26.10.2007
(51) Int. Cl.: A61L 12/08, A61L 27/54, A61K 31/765, A61K 31/79, G02B 1/04

(54) **ANTIMICROBIAL CONTACT LENS AND PROCESSES TO PREPARE ANTIMICROBIAL CONTACT LENSES**
ANTIMIKROBIELLE KONTAKTLINSE UND VERFAHREN ZUR HERSTELLUNG ANTIMIKROBIELLER KONTAKTLINSEN
LENTILLE DE CONTACT ANTIMICROBIENNE ET PROCÉDÉS DE PRÉPARATION DE LENTILLES DE CONTACT ANTIMICROBIENNES

(30) Priority: 31.10.2006 US 863698 P
(43) Date of publication of application: 26.08.2009
(73) Proprietor: Johnson & Johnson Vision Care, Inc., Jacksonville, FL 32256 (US)
(72) Inventor: RATHORE, Osman, Jacksonville, FL 32256 (US); ALVAREZ-CARRIGAN, Nayiby, St. Augustine, FL 32092 (US); BALASUBRAMANIAN, Kanda Kumar, St Augustine, Florida 32092 (US)
(74) Representative: Tunstall, Christopher Stephen
(86) International application number: PCT/US2007/082649
(87) International publication number: WO 2008/073593

(56) References cited:
- WO-A-2004/047878
- WO-A-2004/047879
- WO-A-2006/012000
- DE-A1- 10 024 363
- US-A1- 2003 095 230
- US-A1- 2005 013 842

## Description

### FIELD OF THE INVENTION

This invention relates to methods of preparing antimicrobial lenses

### BACKGROUND OF THE INVENTION

Contact lenses have been used commercially to improve vision since the 1950s. The first contact lenses were made of hard materials. They were used by a patient during waking hours and removed for cleaning. Current developments in the field gave rise to soft contact lenses, which may be worn continuously, for several days or more without removal for cleaning. Although many patients favor these lenses due to their increased comfort, these lenses can cause some adverse reactions to the user. The extended use of the lenses can encourage the buildup of bacteria or other microbes, particularly, *Pseudomonas aeruginosa,* on the surfaces of soft contact lenses. The build-up of bacteria and other microbes can cause adverse side effects such as contact lens acute red eye and the like. Although the problem of bacteria and other microbes is most often associated with the extended use of soft contact lenses, the build-up of bacteria and other microbes occurs for users of hard contact lens wearers as well.

WO2004/047879 and WO2004/047878 disclose ophthalmic devices comprising a metal salt that are substantially free from unwanted haze. US 5,820,918 discloses medical devices made from a water absorbable polymer material with a medical compound having low solubility in aqueous solutions such as an antiseptic or radiopaque compound. However, the procedures disclosed in the examples yield opaque devices which are not suitable for ophthalmic devices such as contact lenses.

Therefore, there is a need to produce contact lenses that inhibit the growth of bacteria or other microbes and/or the adhesion of bacteria or other microbes on the surface of contact lenses. Further there is a need to produce contact lenses which do not promote the adhesion and/or growth of bacteria or other microbes on the surface of the contact lenses. Also there is a need to produce contact lenses that inhibit adverse responses related to the growth of bacteria or other microbes. Still further there is a need to produce the foregoing contact lenses in a manner that produces a lens of clarity suitable to permit a user to clearly see from said lenses. These needs are met by the following invention.

### DETAILED DESCRIPTION OF THE INVENTION

This invention includes a method of preparing an antimicrobial lens comprising a metal salt, wherein said method comprises the steps of
(a) treating a cured lens, with a salt precursor and
(b) treating the lens of step (a) with a dispersing agent and a metal agent.
As used herein, the term, "antimicrobial lens" means a lens that exhibits one or more of the following properties, the inhibition of the adhesion of bacteria or other microbes to the lenses, the inhibition of the growth of bacteria or other microbes on lenses, and the killing of bacteria or other microbes on the surface of lenses or in an area surrounding the lenses. For purposes of this invention, adhesion of bacteria or other microbes to lenses, the growth of bacteria or other microbes on lenses and the presence of bacteria or other microbes on the surface of lenses are collectively referred to as "microbial colonization." Preferably, the lenses of the invention exhibit a reduction of viable bacteria or other microbe of at least about 0.25 log, more preferably at least about 0.5 log, most preferably at least about 1.0 log (≥ 90% inhibition). Such bacteria or other microbes include but are not limited to those organisms found in the eye, particularly *Pseudomonas aeruginosa, Acanthamoeba species, Staphylococcus. aureus, Escherichia. coli, Staphylococcus epidermidis,* and *Serratia marcesens.*

As use herein, the term "metal salt" means any molecule having the general formula [M]ₐ [X]_{b} wherein X contains any negatively charged ion, a is ≥ 1, b is ≥ 1 and M is any positively charged metal selected from, but not limited to, the following Al⁺³, Co⁺², Co⁺³, Ca⁺², Mg⁺², Ni⁺², Ti⁺², Ti⁺³, Ti⁺⁴, V⁺², V⁺³, V⁺⁵, Sr⁺², Fe⁺², Fe⁺³, Ag⁺², Ag⁺¹, Au⁺², Au⁺³, Au⁺¹, Pd⁺², Pd⁺⁴, Pt⁺², Pt⁺⁴, Cu⁺¹, Cu⁺², Mn⁺², Mn⁺³, Mn⁺⁴, Zn⁺², and the like. Examples of X include but are not limited to CO₃⁻², NO₃⁻¹, PO₄⁻³, Cl⁻¹, I⁻¹, Br⁻¹, S⁻², O⁻² and the like. Further X includes negatively charged ions containing CO₃⁻² NO₃⁻¹, PO4⁻³, Cl⁻¹, I⁻¹, Br⁻¹, S⁻², O⁻², and the like, such as C₁₋₅alkylCO₂⁻¹. As used herein the term metal salts does not include zeolites, disclosed in WO03/011351. The preferred a is 1, 2, or 3. The preferred b is 1, 2, or 3. The preferred metals ions are Mg⁺², Zn⁺², Cu⁺¹, Cu⁺², Au⁺², Au⁺³, Au⁺¹, Pd⁺², Pd⁺⁴, Pt⁺², Pt⁺⁴, Ag⁺², and Ag⁺¹. The particularly preferred metal ion is Ag⁺¹. Examples of suitable metal salts include but are not limited to manganese sulfide, zinc oxide, zinc sulfide, copper sulfide, and copper phosphate. Examples of silver salts include but are not limited to silver nitrate, silver sulfate, silver iodate, silver carbonate, silver phosphate, silver sulfide, silver chloride, silver bromide, silver iodide, and silver oxide. The preferred silver salts are silver iodide, silver chloride, and silver bromide. The lenses of the invention are ophthalmic lenses (a detailed description of these lenses follows) and the clarity of the lenses is of concern to users. In order to produce lenses having a clarity suitable for ophthalmic purposes, it is preferred that the diameter of the metal salt particles is less than about ten microns (10 µm), more preferably less than about 1 µm, even more preferably less than about 400 nm. Particle size of the metal salt in the antimicrobial lens may be determined by the following test.

The samples for scanning electron microscopy ("SEM") were prepared for profile analyses by mounting the whole lens vertically in a 25 mm diameter aluminum holder that had been cut in half and drilled and tapped for two machine screws to clamp the specimen. The lens was clamped so that half of the material was above the surface of the holder. A clean single edge razor was then used to slice the lens in half in one smooth stroke to avoid tearing the cut surface. These samples were then carbon coated in a vacuum evaporator to ensure conductivity. The far edge of these samples was daubed with colloidal carbon paint for better conductivity.

Samples were prepared for surface analyses by taking the remaining half of the lens and slicing a strip from near the diameter that was then carefully placed on a 25 mm diameter holder, with two double sided carbon "sticky tabs" on the top surface, with the concave surface up. Lens surfaces were also analyzed on the convex surface by mounting the remaining chord of lens material convex side up also on two "sticky tabs". In both cases, a sheet of clean Teflon material (.032" thick) was used to press the contact lens flat to the carbon "sticky tabs". These samples were also coated with 20-40 nm of Spec-Pure graphite in a carbon vacuum evaporator. The far edge of these samples was daubed with colloidal carbon paint for better conductivity.

Three images (left, middle and right) were taken from both convex and concave surfaces of each lens at various magnifications. Profile images taken at magnifications, 5000 x and 12,500x. For each position (left, middle or right) of the lens piece, about 5 to 10 images were taken starting at the convex end of the lens to the concave end depending upon the thickness of the lens. The images were "stitched" together to obtain the silver iodide particle size and distribution information inside the lens.

Particle size distribution measurements for both surface and profiles were extracted from 5000x images using Scion Image analysis software. The results were compiled from three lenses of each lot.

All the images were taken with 5kV beam energy. Though both secondary electron (SE) and back scattered electron (BSE) images were obtained, only BSE images at 5000x were used for particle size analysis due to high contrast obtained for the silver iodide particles compared to the background.

The amount of metal in the lenses is measured based upon the total weight of the lenses. When the metal is silver, the preferred amount of silver is about 0.00001 weight percent (0.1 ppm) to about 10.0 weight percent, preferably about 0.0001 weight percent (1 ppm) to about 1.0 weight percent, most preferably about 0.001 weight percent (10 ppm) to about 0.1 weight percent, based on the dry weight of the lens. With respect to adding metal salts, the molecular weight of the metal salts determines the conversion of weight percent of metal ion to metal salt. The preferred amount of silver salt is about 0.00003 weight percent (0.3 ppm) to about 30.0 weight percent, preferably about 0.0003 weight percent (3 ppm) to about 3.0 weight percent, most preferably about 0.003 weight percent (30 ppm) to about 0.3 weight percent, based on the dry weight of the lens.

The term "salt precursor" refers to any compound or composition that contains a cation that may be substituted with metal ions. The concentration of salt precursor in its solution is between about 0.00001 to about 10.0 weight percent, (0.1 -100,000 ppm) more preferably about 0.0001 to about 1.0 weight percent, (1-10,000 ppm) most preferably about 0.001 to about 0.1 weight percent (10-1000 ppm) based upon the total weight of the solution. Examples of salt precursors include but are not limited to inorganic molecules such as sodium chloride, sodium iodide, sodium bromide, sodium sulfide, lithium chloride, lithium iodide, lithium bromide, lithium sulfide, potassium bromide, potassium chloride, potassium sulfide, potassium iodide, rubidium iodide, rubidium bromide, rubidium chloride, rubidium sulfide, caesium iodide, caesium bromide, caesium chloride, caesium sulfide, calcium chloride, calcium bromide, calcium iodide, calcium sulfide, magnesium chloride, magnesium bromide, magnesium iodide, magnesium sulfide, sodium tetrachloro argentate, and the like. Examples of organic molecules include but are not limited to tetra-alkyl ammonium lactate, tetra-alkyl ammonium sulfate, quaternary ammonium halides, such as tetra-alkyl ammonium chloride, bromide or iodide. The preferred salt precursor is selected from the group consisting of sodium chloride, sodium iodide, sodium bromide, lithium chloride, lithium sulfide, sodium sulfide, potassium sulfide, potassium iodide, and sodium tetrachloro argentite and the particularly preferred salt precursor is sodium iodide.

The term "metal agent" refers to any composition (including aqueous solutions) containing metal ions. Examples of such compositions include but are not limited to aqueous or organic solutions of silver nitrate, silver triflate, or silver acetate, silver tetrafluoroborate, silver sulfate, zinc acetate, zinc sulfate, copper acetate, and copper sulfate, where the concentration of metal agent in solution is about 1µg/mL or greater. The preferred metal agent is aqueous silver nitrate, where the concentration of silver nitrate is the solution is about greater than or equal to 0.0001 to about 2 weight percent (1 ppm-20,000 ppm), more preferably about greater than 0.001 to about 0.1 weight percent (10 ppm-1,000 ppm) based on the total weight of the solution. The term "treating" refers to any method of contacting the metal agent or salt precursor with the lens, where the preferred method is immersing the lens in a solution of the metal agent or the salt precursor. Treating can include heating the lens in a solution of the metal agent or the salt precursor, but it preferred that treating is carried out at ambient temperatures. The time of this treatment can last anywhere from about 30 seconds to about 24 hours, preferably from about 30 seconds to about 15 minutes.

As used herein, the term "dispersing agent" refers to a composition that may be used modulate the interaction between polymers and particles, particularly metal salts that are admixed with such polymers. Examples of dispersing agents include but are not limited to polyvinylpyrrolidone ("PVP"), polyvinylalcohol ("PVA") and derivatives, glycerine, and polyethylene oxide ("PEO"). Other dispersing agents that may be used are nitrogen-containing polymers such as but not limited to poly(dimethyl acrylamide), poly(N-vinyl-N-methylacetamide). Certain non-polymeric materials containing nitrogen and/or sulfur may be used as dispersing agents as well, such as cysteine, methionine, sodium sulfide, sodium thiosulfate, sodium thiocyanate. The particularly preferred dispersing agent is PVP. A variety of weights of PVP are commercially available. The K system is used to distinguish one molecular weight of PVP from another. The preferred K value is K90. It is preferred that the dispersing agent and the metal agent are mixed together with a suitable solvent, such as water, deionized water, alcohols and mixtures thereof, to produce a clear solution of those components. If the metal agent is contained within an aqueous solution, the preferred amount of dispersing agent in the solution is about 0.1 % to about 50%, more preferably about 4% to about 10%, even more preferably about 2.5% to about 6%, most preferably about 5%. In some embodiments the molar ratio of dispersing agent unit to metal agent is at least about 1.5, at least about 2, and in some embodiments at least about 4.

It is believed that the dispersing agent in the metal agent solution forms a complex with the metal agent. In this embodiment, it is desirable to allow the metal agent to fully complex with the dispersing agent prior to combining the metal agent solution with the cured lens. "Fully complexed" means that substantially all the metal ions have complexed with at least one dispersing agent. "Substantially all" means at least about 90%, and in some embodiments at least about 95% of said metal ions have complexed with at least one dispersing agent.

The complex-forming time may be monitored in solution via spectroscopy, such as via UV-VIS or FTIR. The spectra of the metal agent solution without the dispersing agent is measured. The spectra of the metal agent solution is monitored after addition of the dispersing agent, and the change in spectra is monitored. The complex-forming time is the time at which the spectral change plateaus.

Alternatively, complexation time may be measured empirically by forming a series of metal agent-dispersing agent solutions having the same concentration, allowing each solution to mix for a different time and mixing each metal agent-dispersing agent solution batch-wise with the salt precursor solution. The metal agent-dispersing agent solutions which are mixed for complex-forming times will form clear solutions when the metal agent and salt precursor solutions are poured together directly without controlling the rate of addition.

Complexation conditions include complexation time (discussed above), temperature, ratio of the dispersing agent to the metal agent and stirring rates. Increasing the temperature, molar ratio of dispersing agent to metal agent and stirring rate, will decrease complexation time. Those of skill in the art will, with reference to the teachings herein, can vary the conditions to achieve the disclosed complexation levels.

As used herein, the term "lens" refers to an ophthalmic device that resides in or on the eye. These devices can provide any of all of the following effects, optical correction, wound care, drug delivery, diagnostic functionality, cosmetic enhancement, and the like. The term lens includes but is not limited to soft contact lenses, hard contact lenses, intraocular lenses, overlay lenses, ocular inserts, and optical inserts. Soft contact lenses are made from silicone elastomers or hydrogels, which include but are not limited to silicone hydrogels, and fluorohydrogels.

For example the term lens includes but is not limited to those made from the soft contact lens formulations described in US 5,710,302, WO 9421698, EP 406161, JP 2000016905, U.S. 5,998,498, US Pat. App. No. 09/532,943, U.S. 6,087,415, U.S. 5,760,100, U.S.5,776,999, U.S. 5,789,461, U.S. 5,849,811, and U.S. 5,965,631. In addition, metal salts of the invention may be added to commercial soft contact lenses. Examples of soft contact lenses formulations include but are not limited to the formulations of etafilcon A, genfilcon A, lenefilcon A, polymacon, acquafilcon A, balafilcon A, galyfilcon A, senofilcon A and lotrafilcon A. The preferable lens formulations are etafilcon A, balafilcon A, acquafilcon A, galyfilcon A, lotrafilcon A, and silicone hydrogels, as prepared in U.S. 5,998,498, US Ser. No. 09/532,943, a continuation-in-part of US Pat App. No. 09/532,943, filed on August 30, 2000, WO03/22321, U.S. 6,087,415, U.S. 5,760,100, U.S. 5,776,999, U.S. 5,789,461, U.S. 5,849,811, and U.S. 5,965,631.

Preferably the metal salts are added to lenses made from silicone hydrogel components, A silicone-containing component is one that contains at least one [-Si-O-Si] group, in a monomer, macromer or prepolymer. Preferably, the Si and attached O are present in the silicone-containing component in an amount greater than 20 weight percent, and more preferably greater than 30 weight percent of the total molecular weight of the silicone-containing component, Useful silicone-containing components preferably comprise polymerizable functional groups such as acrylate, methacrylate, acrylamide, methacrylamide, N-vinyl lactam, N-vinylamide, and styryl functional groups. Examples of silicone components which may be included in the silicone hydrogel formulations include, but are not limited to silicone macromers, prepolymers and monomers. Examples of silicone macromers include, without limitation, polydimethylsiloxane methacrylated with pendant hydrophilic groups as described in United States Patents Nos. 4,259,467; 4,260,725 and 4,261,875; polydimethylsiloxane macromers with polymerizable functional group(s) described in U.S. Patents Nos. 4,136,250; 4,153,641; 4,189,546; 4,182,822; 4,343,927; 4,254,248; 4,355,147; 4,276.402; 4,327,203; 4,341,889; 4,486,577; 4,605,712; 4,543,398; 4,661,575; 4,703,097; 4,837,289; 4,954,586; 4,954,587; 5,346,946; 5,358,995; 5,387,632 ; 5,451,617; 5,486,579; 5,962,548; 5,981,615; 5,981,675; and 6,039,913; polysiloxane macromers incorporating hydrophilic monomers such as those described in U.S. Patents Nos. 5,010,141; 5,057,578; 5,314,960; 5,371,147 and 5,336,797; macromers comprising polydimethylsiloxane blocks and polyether blocks such as those described in U.S. Patents Nos. 4,871,785 and 5,034,461, combinations thereof and the like.

The silicone and/or fluorine containing macromers described in U.S. Patents Nos. 5,760,100; 5,776,999; 5,789,461; 5,807,944; 5,965,631 and 5,958,440 may also be used. Suitable silicone monomers include tris(trimethylsiloxy)silylpropyl methacrylate, hydroxyl functional silicone containing monomers, such as 3-methacryloxy-2-hydroxypropyloxy)propylbis(trimethylsiloxy)methylsilane and those disclosed in WO03/22321, and mPDMS containing or the siloxane monomers described in U.S. Patents Nos. 4,120,570, 4,139,692, 4,463,149, 4,450,264, 4,525,563; 5,998,498; 3,808,178; 4,139,513; 5,070,215; 5,710,302; 5,714,557 and 5,908,906.

Additional suitable siloxane containing monomers include, amide analogs of TRIS described in U.S. 4,711,943, vinylcarbamate or carbonate analogs decribed in U.S. 5,070,215, and monomers contained in U.S. 6,020,445, monomethacryloxypropyl terminated polydimethylsiloxanes, polydimethylsiloxanes, 3-methacryloxypropylbis(trimethylsiloxy)methylsilane, methacryloxypropylpentamethyl disiloxane and combinations thereof.

In addition to soft contact lens formulations, hard contact lenses may be used. Examples of hard contact lens formulations are made from polymers that include but are not limited to polymers of polymethylmethacrylate, silicon acrylates, silicone acrylates, fluoroacrylates, fluoroethers, polyacetylenes, and polyimides, where the preparation of representative examples may be found in JP 200010055, JP 6123860 and U.S. Patent 4,330,383. Intraocular lenses of the invention can be formed using known materials. For example, the lenses may be made from a rigid material including, without limitation, polymethyl methacrylate, polystyrene, polycarbonate, or the like, and combinations thereof. Additionally, flexible materials may be used including, without limitation, hydrogels, silicone materials, acrylic materials, fluorocarbon materials and the like, or combinations thereof. Typical intraocular lenses are described in WO 0026698, WO 0022460, WO 9929750, WO 9927978, WO 0022459, and JP 2000107277. U.S. 4,301,012; 4,872,876; 4,863,464; 4,725,277; 4,731,079.

It has been found that when the metal salt is incorporated in accordance with the teachings of the present invention, ophthalmic devices that are substantially free from unwanted haze are produced. Preferably, the lenses of the invention are optically clear, with optical clarity comparable to lenses such as lenses made from etafilcon A, genfilcon A, galyfilcon A, lenefilcon A, polymacon, acquafilcon A, balafilcon A, and lotrafilcon A. Specifically, lenses of the present invention have a percent haze that is less than about 200%, preferably less than about 150%, more preferably less than about 100%, even more preferably less than 30%, even more preferably, between less than about 30% and about 9%.

The percentage of haze is measured using the following method. A hydrated test lens in borate buffered saline (SSPS) is placed in a clear 20 x 40 x 10 mm glass cell at ambient temperature above a flat black background, illuminating from below with a fiber optic lamp (Titan Tool Supply Co. fiber optic light with 0.5" diameter light guide set at a power setting of 4-5.4) at an angle 66° normal to the lens cell, and capturing an image of the lens from above, normal to the lens cell with a video camera (DVC 1300C:19130 RGB camera with Navitar TV Zoom 7000 zoom lens) placed 14 mm above the lens platform. The background scatter is subtracted from the scatter of the lens by subtracting an image of a blank cell using EPIX XCAP V 1.0 software. The subtracted scattered light image is quantitatively analyzed, by integrating over the central 10 mm of the lens, and then comparing to a -1.00 diopter CSI Thin Lens®, which is arbitrarily set at a haze value of 100, with no lens set as a haze value of 0. Five lenses are analyzed and the results are averaged to generate a haze value as a percentage of the standard CSI lens.

The term "cured" refers to any of a number of methods used to react a mixture of lens components (ie, momoner, prepolymers, macromers and the like) to form lenses. Lenses can be cured by light or heat. The preferred method of curing is with radiation, preferably UV or visible light, and most preferably with visible light. The lens formulations of the present invention can be formed by any of the methods known to those skilled in the art, such as shaking or stirring, and used to form polymeric articles or devices by known methods.

For example, the antimicrobial lenses of the invention may be prepared by mixing reactive components and any diluent(s) with a polymerization initator and curing by appropriate conditions to form a product that can be subsequently formed into the appropriate shape by lathing, cutting and the like. Alternatively, the reaction mixture may be placed in a mold and subsequently cured into the appropriate article.

Various processes are known for processing the lens formulation in the production of contact lenses, including spincasting and static casting. Spincasting methods are disclosed in U.S. 3,408,429 and 3,660,545, and static casting methods are disclosed in U.S. 4,113,224 and 4,197,266. The preferred method for producing antimicrobial lenses of this invention is by molding. In the case of hydrogel lenses, for this method, the lens formulation is placed in a mold having the approximate shape of the final desired lens, and the lens formulation is subjected to conditions whereby the components polymerize, to produce a hardened disc that is subjected to a number of different processing steps including treating the polymerized lens with liquids (such as water, inorganic salts, or organic solutions) to swell, or otherwise equilibrate this lens prior to enclosing the lens in its final packaging. These methods are further described in U.S. Pat. Nos. 4,495,313; 4,680,336; 4,889,664; and 5,039,459. Polymerized lenses that have not been swelled or otherwise equilibrated are considered cured lenses for purposes of this invention.

Further, the invention includes a method of preparing an antimicrobial lens comprising, consisting essentially of, or consisting of a metal salt, wherein the method comprises, consists essentially of, or consists of the steps of
(a) treating a cured lens with a metal agent and a dispersing agent;
(b) treating the lens of step (a) with a salt precursor.
The terms antimicrobial lens, metal salt, salt precursor, metal agent, dispersing agent, and treating all have their aforementioned meanings and preferred ranges.

Still further, the invention includes a method of preparing an antimicrobial lens comprising, consisting essentially of, or consisting of a metal salt, wherein the method comprises, consists essentially of, or consists of the steps of
(a) treating a cured lens with a metal agent and a dispersing agent; and
(b) treating the lens of step (a) with a salt precursor and a dispersing agent;
The terms antimicrobial lens, metal salt, salt precursor, metal agent, dispersing agent and treating all have their aforementioned meanings and preferred ranges. The dispersing agents of steps (a) and (b) can be the same or different, however, it is preferred that they are the same.

Yet still further, invention includes a method of preparing an antimicrobial lens comprising, consisting essentially of, or consisting of a metal salt, wherein said method comprises, consists essentially of, or consists of the steps of
(a) treating a cured lens, with a salt precursor and a dispersing agent and
(b) treating the lens of step (a) with a dispersing agent and a metal agent.
The terms antimicrobial lens, metal salt, salt precursor, metal agent, dispersing agent and treating all have their aforementioned meanings and preferred ranges. The dispersing agents of steps (a) and (b) can be the same or different, however, it is preferred that they are the same.

Still further the invention include an antimicrobial lens comprising, consisting essentially of, or consisting of a metal salt, made by a method, wherein said method comprises, consists essentially of, or consists of the steps of
(a) treating a cured lens with a metal agent and a dispersing agent, and
(b) treating the lens of step (a) with a salt precursor;
The terms antimicrobial lens, metal salt, salt precursor, metal agent, and treating all have their aforementioned meanings and preferred ranges.

Yet still further the invention includes an antimicrobial lens comprising, consisting essentially of, or consisting of a metal salt made by a method, wherein said method comprises, consists essentially of, or consists of the steps of
(a) treating a cured lens, a salt precursor; and
(b) treating the lens of step (a) with a dispersing agent and a metal agent.
The terms antimicrobial lens, metal salt, salt precursor, metal agent, dispersing agent and treating all have their aforementioned meanings and preferred ranges.

Still further, the invention includes an antimicrobial lens comprising, consisting essentially of, or consisting of a metal salt made by a method, wherein said method comprises, consists essentially of, or consists of the steps of
(a) treating a cured lens with a metal agent and a dispersing agent; and
(b) treating the lens of step (a) with a salt precursor and a dispersing agent;
The terms antimicrobial lens, metal salt, salt precursor, metal agent, dispersing agent and treating all have their aforementioned meanings and preferred ranges.

The dispersing agents of steps (a) and (b) can be the same or different, however, it is preferred that they are the same.

Yet still further, invention includes an antimicrobial lens comprising, consisting essentially of, or consisting of a metal salt made by a method, wherein said method comprises, consists essentially of, or consists of the steps of
(a) treating a cured lens, with a salt precursor and a dispersing agent and
(b) treating the lens of step (a) with a dispersing agent and a metal agent.
The terms antimicrobial lens, metal salt, salt precursor, metal agent, dispersing agent and treating all have their aforementioned meanings and preferred ranges. The dispersing agents of steps (a) and (b) can be the same or different, however, it is preferred that they are the same.

Although haze is one measurement of the clarity of a lens, a lens can have low overall clarity, but can contain localized areas of deposited metal agents ("localized areas of deposition"). One of the advantages of the lenses of the invention and the methods to produce them is a reduction in the localized areas of deposition. This can be demonstrated by dark field microscopy according the following methods.

The hydrated test lens to be inspected is placed in a crystallization dish from Kimble Glass, Inc. [KIMAX 23000 5035, 50x35mm]. Borate buffered sodium sulfate solution (SSPS, 10-12 mL) filtered through a ≤0.45um filter is added to the dish. The lens is placed close to the center of the dish to minimize artifacts in the image resulting from reflected light. A Nikon SMZ 1500 microscope is used for the test. The dish containing the lens is placed on the light stage. The light source is set to the highest intensity, and the microscope is set in D.F. (Dark Field) mode. The light aperture on the microscope is completely opened. The software used to capture the images is called 'Aquinto made by http://www.olympus-sis.com/', (formerly known as Aquinto). A Nikon DXM1200F digital camera is used to capture images with the following camera settings (set in Program Aquinto): 'Exposure time' = 53.0555 ms, 'Color Filter' = 'gray', 'Capture Mode' = '960x768','Mirror horz', 'Mirror vert', 'Logarithmic', and 'Auto refresh' are deselected. Under the' Optimize' tab (in Program Aquinto) all filter settings are set to 'No filter'. The captured images are evaluated to look for areas of localized deposition.

In order to illustrate the invention the following examples are included. These examples do not limit the invention. They are meant only to suggest a method of practicing the invention. Those knowledgeable in contact lenses as well as other specialties may find other methods of practicing the invention. However, those methods are deemed to be within the scope of this invention.

### EXAMPLES

The following abbreviations were used in the examples

### Sodium Sulfate packing solution (SSPS)

SSPS contains the following in deionized H₂O:
1.40 weight % sodium sulfate
0.185 weight % sodium borate [*1330-43-4*]*,* Mallinckrodt
0.926 weight % boric acid [*10043-35-3*]*,* Mallinckrodt
0.005 weight % methylcellulose

### Example 1

### Preparation of Antimicrobial Lenses From Cured Lenses

Cured and hydrated galyfilcon A lenses are placed in a jar with sodium iodide solution in deionized water, containing 50ppm of methylcellulose (∼3mL solution per lens,) and rolled on a jar roller overnight. The lenses were transferred from the jar to a blister pack where the excess sodium iodide solution was removed. A solution (800 µL) of silver nitrate in deionized water, containing the appropriate dispersion agent, was added to the blister for two to five minutes. The silver nitrate solution was removed, and the lenses were placed in a jar containing deionized water and rolled on a jar roller for approximately thirty minutes. The deionized water was replaced with borate buffered sodium sulfate solution containing 50 ppm methylcellulose in water (SSPS), and allowed to roll on the jar roller for an additional 30 minutes. The solution was then replaced with fresh SSPS.

The lenses were then transferred to new blisters and dosed with 950 µL of SSPS. The blisters were sealed and autoclaved at 125°C for 18 minutes and analyzed for haze using the methods described herein and silver content using the methods described below. The results are presented in Table 1. This data shows that the addition of dispersion agents reduces the Haze% or improves lens-to-lens haze uniformity, as demonstrated by reduced standard deviation.

Silver content of the lenses after lens autoclaving was determined by Instrumental Neutron Activation Analysis "INAA". INAA is a qualitative and quantitative elemental analysis method based on the artificial induction of specific radionuclides by irradiation with neutrons in a nuclear reactor. Irradiation of the sample is followed by the quantitative measurement of the characteristic gamma rays emitted by the decaying radionuclides. The gamma rays detected at a particular energy are indicative of a particular radionuclide's presence, allowing for a high degree of specificity. Becker, D. A.; Greenberg, R.R.; Stone, S. F. J. Radioanal. Nucl. Chem. 1992, 160(1), 41-53; Becker, D. A.; Anderson, D. L.; Lindstrom, R. M.; Greenberg, R. R.; Garrity, K. M.; Mackey, E. A. J. Radioanal. Nucl. Chem. 1994, 179(1), 149-54. The INAA procedure used to quantify silver content in contact lens material uses the following two nuclear reactions:
1. In the activation reaction, ¹¹⁰Ag is produced from stable ¹⁰⁹Ag (isotopic abundance = 48.16 %) after capture of a radioactive neutron produced in a nuclear reactor.
2. In the decay reaction, ¹¹⁰Ag (*τ*^{1/2} = 24.6 seconds) decays primarily by negatron emission proportional to initial concentration with an energy characteristic to this radio- nuclide (657.8 keV).
The gamma-ray emission specific to the decay of ¹¹⁰Ag from irradiated. standards and samples are measured by gamma-ray spectroscopy, a well-established pulse-height analysis technique, yielding a measure of the concentration of the analyte.

**Table 1**

| % dispersion agent | Nal ppm | AgNO₃ soak time (min) | AgNO₃ ppm | Ag (µg) | Std. Dev (µg). | Haze (% vs CSI) | Std. Dev (% vs. CSI). |
|---|---|---|---|---|---|---|---|
| none | 1100 | 2 | 700 | 17.8 | 0.2 | 42.3 | 14.0 |
| none | 1100 | 2 | 700 | 18.8 | 0.3 | 48.4 | 11.0 |
| none | 1100 | 2 | 700 | 15.8 | 2.1 | 22 | 5.74 |
| 1%PVP K-90 | 1100 | 2 | 700 | 17.8 | 0.7 | 23.3 | 0.8 |
| 1%PVP K-90 | 1100 | 2 | 700 | 17.8 | 0.6 | 22.7 | 1.4 |
| 2.5%PVP K-90 | 1500 | 3 | 950 | 24.1 | 0.8 | 24.0 | 1.3 |
| 2.5%PVP K-90 | 1500 | 3 | 950 | 23.8 | 0.5 | 21.5 | 1.1 |
| 2.5%PVP K-90 | 1100 | 3 | 700 | 16.3 | 2.5 | 22.7 | 1.0 |
| 2.5%PVP K-90 | 1100 | 3 | 700 | 17.1 | 0.4 | 23.4 | 1.1 |
| 5%PVP K-90 | 1100 | 3 | 700 | 17.8 | 1.8 | 22.7 | 1.2 |
| 5%PVP K-90 | 1100 | 3 | 700 | 18.2 | 1.3 | 23.5 | 1.0 |
| 5% PVP K-12 | 1100 | 3 | 700 | 16.7 | 1.1 | 18.4 | 1.5 |
| 10% PVP K-12 | 1100 | 3 | 700 | 16.5 | 0.5 | 14.1 | 1.3 |
| 15% PVP K-12 | 1100 | 3 | 700 | 17.0 | 1.3 | 14 | 1.8 |
| 5% PEO 10K | 1100 | 3 | 700 | 17.9 | 1.5 | 18.8 | 3.4 |
| 10% PEO 10K | 1100 | 3 | 700 | 17.4 | 1.7 | 22 | 6.1 |
| 25% GLY | 1100 | 3 | 700 | 17.4 | 0.4 | 28.6 | 5.4 |
| 6% PVA 40K | 1100 | 3 | 700 | 18.0 | 1.3 | 26.5 | 5.4 |
| 4% PVA 120K | 1100 | 3 | 700 | 17.9 | 0.7 | 17.4 | 2.6 |
| 5%PVP K-90 | 1100 | 3 | 700 | 18.0 | 1.2 | 14.2 | 1.6 |

| | | | | | | | |
|---|---|---|---|---|---|---|---|
| Abbreviations PVA is polyvinylalcohol, PEO is polyethylene oxide, GLY is glycerine, PVP is polyvinylpyrrolidone | | | | | | | |

## Claims

1. A method of preparing an antimicrobial lens comprising a metal salt, wherein said method comprises the steps of
(a) treating a cured lens, with a salt precursor and
(b) treating the lens of step (a) with a dispersing agent and a metal agent.

2. A method of preparing an antimicrobial lens comprising a metal salt, wherein the method comprises the steps of
(a) treating a cured lens with a metal agent and a dispersing agent;
(b) treating the lens of step (a) with a salt precursor.

3. The method of claim 1 or 2 wherein the dispersing agent is selected from the group consisting of polyvinylpyrrolidone, polyvinylalcohol, glycerine, polyethylene oxide, PVP K-12, PVP K-30, PVP K-60, and PVP K-90.

4. The method of claim 1 or 2 wherein the salt precursor is selected from the group consisting of sodium chloride, sodium iodide, sodium bromide, lithium chloride, lithium sulfide, potassium iodide, sodium sulfide, potassium sulfide, sodium tetrachloro argentite, tetra-alkyl ammonium lactate, tetra-alkyl ammonium sulfate, tetra-alkyl ammonium chloride, tetra-alkyl ammonium bromide and tetra-alkyl ammonium iodide.

5. The method claim 1 wherein the metal agent is selected from the group consisting of silver tetrafluoroborate, silver sulfate, zinc acetate, zinc sulfate, copper acetate, copper sulphate, and silver nitrate,

6. The method of claim 2 wherein the metal agent is selected from the group consisting of silver nitrate, silver triflate, silver acetate, silver tetrafluoroborate, silver sulfate, zinc acetate, zinc sulfate, copper acetate, and copper sulfate.

7. The method of claim 1 or 2 wherein the metal salt is selected from the group consisting of manganese sulfide, zinc oxide, zinc sulfide, copper sulfide, copper phosphate, silver nitrate, silver sulfate, silver iodate, silver carbonate, silver phosphate, silver sulfide, silver chloride, silver bromide, silver iodide, and silver oxide.

8. The method of claim 2, wherein step (b) comprises treating the lens of step (a) with a salt precursor and a dispersing agent.

9. The method of claim 1, wherein step (a) comprises treating a cured lens with a salt precursor and a dispersing agent.

10. An antimicrobial lens comprising a metal salt obtainable by the method according to any one of the preceding claims.

## Patentansprüche

1. Verfahren zur Herstellung einer antimikrobiellen Linse, umfassend ein Metallsalz, wobei das Verfahren die Schritte umfasst:
(a) Behandeln einer gehärteten Linse mit einem Salzvorläufer und
(b) Behandeln der Linse aus Schritt (a) mit einem Dispergiermittel und einem Metallmittel.

2. Verfahren zur Herstellung einer antimikrobiellen Linse, umfassend ein Metallsalz, wobei das Verfahren die Schritte umfasst:
(a) Behandeln einer gehärteten Linse mit einem Metallmittel und einem Dispergiermittel;
(b) Behandeln der Linse aus Schritt (a) mit einem Salzvorläufer.

3. Verfahren nach Anspruch 1 oder 2, wobei das Dispergiermittel ausgewählt ist aus der Gruppe bestehend aus Polyvinylpyrrolidon, Polyvinylalkohol, Glycerin, Polyethylenoxid, PVP K-12, PVP K-30, PVP K-60 und PVP K-90.

4. Verfahren nach Anspruch 1 oder 2, wobei der Salzvorläufer ausgewählt ist aus der Gruppe bestehend aus Natriumchlorid, Natriumiodid, Natriumbromid, Lithiumchlorid, Lithiumsulfid, Kaliumiodid, Natriumsulfid, Kaliumsulfid, Natriumtetrachlorargentit, Tetraalkylammoniumlactat, Tetraalkylammoniumsulfat, Tetraalkylammoniumchlorid, Tetraalkylammoniumbromid und Tetraalkylammoniumiodid.

5. Verfahren nach Anspruch 1, wobei das Metallmittel ausgewählt ist aus der Gruppe bestehend aus Silbertetrafluorborat, Silbersulfat, Zinkacetat, Zinksulfat, Kupferacetat, Kupfersulfat und Silbernitrat.

6. Verfahren nach Anspruch 2, wobei das Metallmittel ausgewählt ist aus der Gruppe bestehend aus Silbernitrat, Silbertriflat, Silberacetat, Silbertetrafluorborat, Silbersulfat, Zinkacetat, Zinksulfat, Kupferacetat und Kupfersulfat.

7. Verfahren nach Anspruch 1 oder 2, wobei das Metallsalz ausgewählt ist aus der Gruppe bestehend aus Mangansulfid, Zinkoxid, Zinksulfid, Kupfersulfid, Kupferphosphat, Silbernitrat, Silbersulfat, Silberiodat, Silbercarbonat, Silberphosphat, Silbersulfid, Silberchlorid, Silberbromid, Silberiodid und Silberoxid.

8. Verfahren nach Anspruch 2, wobei Schritt (b) Behandeln der Linse aus Schritt (a) mit einem Salzvorläufer und einem Dispergiermittel umfasst.

9. Verfahren nach Anspruch 1, wobei Schritt (a) Behandeln einer gehärteten Linse mit einem Salzvorläufer und einem Dispergiermittel umfasst.

10. Antimikrobielle Linse, umfassend ein Metallsalz, erhältlich nach dem Verfahren gemäß einem der vorhergehenden Ansprüche.

## Revendications

1. Procédé de préparation d'une lentille antimicrobienne comprenant un sel métallique, ledit procédé comprenant les étapes consistant à
(a) traiter une lentille durcie, avec un précurseur de sel et
(b) traiter la lentille de l'étape (a) avec un agent dispersant et un agent métallique.

2. Procédé de préparation d'une lentille antimicrobienne comprenant un sel métallique, le procédé comprenant les étapes consistant à
(a) traiter une lentille durcie avec un agent métallique et un agent dispersant ;
(b) traiter la lentille de l'étape (a) avec un précurseur de sel.

3. Procédé selon la revendication 1 ou 2 dans lequel l'agent dispersant est choisi dans le groupe constitué par la polyvinylpyrrolidone, le poly(alcool vinylique), la glycérine, le poly(oxyde d'éthylène), la PVP K-12, la PVP K-30, la PVP K-60 et la PVP K-90.

4. Procédé selon la revendication 1 ou 2 dans lequel le précurseur de sel est choisi dans le groupe constitué par le chlorure de sodium, l'iodure de sodium, le bromure de sodium, le chlorure de lithium, le sulfure de lithium, l'iodure de potassium, le sulfure de sodium, le sulfure de potassium, le tétrachloroargentite de sodium, un lactate de tétraalkylammonium, un sulfate de tétraalkylammonium, un chlorure de tétraalkylammonium, un bromure de tétraalkylammonium et un iodure de tétraalkylammonium.

5. Procédé selon la revendication 1 dans lequel l'agent métallique est choisi dans le groupe constitué par le tétrafluoroborate d'argent, le sulfate d'argent, l'acétate de zinc, le sulfate de zinc, l'acétate de cuivre, le sulfate de cuivre et le nitrate d'argent.

6. Procédé selon la revendication 2 dans lequel l'agent métallique est choisi dans le groupe constitué par le nitrate d'argent, le triflate d'argent, l'acétate d'argent, le tétrafluoroborate d'argent, le sulfate d'argent, l'acétate de zinc, le sulfate de zinc, l'acétate de cuivre et le sulfate de cuivre.

7. Procédé selon la revendication 1 ou 2 dans lequel le sel métallique est choisi dans le groupe constitué par le sulfure de manganèse, l'oxyde de zinc, le sulfure de zinc, le sulfure de cuivre, le phosphate de cuivre, le nitrate d'argent, le sulfate d'argent, l'iodate d'argent, le carbonate d'argent, le phosphate d'argent, le sulfure d'argent, le chlorure d'argent, le bromure d'argent, l'iodure d'argent et l'oxyde d'argent.

8. Procédé selon la revendication 2, dans lequel l'étape (b) comprend le traitement de la lentille de l'étape (a) avec un précurseur de sel et un agent dispersant.

9. Procédé selon la revendication 1, dans lequel l'étape (a) comprend le traitement d'une lentille durcie avec un précurseur de sel et un agent dispersant.

10. Lentille antimicrobienne comprenant un sel métallique pouvant être obtenue par le procédé selon l'une quelconque des revendications précédentes.
